# EUROPEAN PATENT SPECIFICATION

(11) **EP 2 540 247 B1**
(45) Date of publication and mention of the grant of the patent: **27.04.2016**
(21) Application number: 11747582.2
(22) Date of filing: 25.02.2011
(51) Int. Cl.: A61B 18/20, A61N 5/06

(54) **CATHETER FOR PHOTODYNAMIC ABLATION OF CARDIAC MUSCLE TISSUE**
KATHETER ZUR PHOTODYNAMISCHEN ABLATION VON HERZMUSKELGEWEBE
CATHÉTER POUR UNE ABLATION PHOTODYNAMIQUE DU TISSU MUSCULAIRE CARDIAQUE

(30) Priority: 26.02.2010 JP 2010042669
(43) Date of publication of application: 02.01.2013
(73) Proprietor: Keio University, Tokyo 108-8345 (JP)
(72) Inventor: ARAI, Tsunenori, Yokohama-shi Kanagawa 223-8522 (JP); ITO, Arisa, Yokohama-shi Kanagawa 223-8522 (JP)
(74) Representative: Hodsdon, Stephen James
(86) International application number: PCT/JP2011/055004
(87) International publication number: WO 2011/105631

(56) References cited:
- WO-A1-2008/066206
- WO-A1-2009/072060
- JP-A- 11 332 876
- JP-A- 2003 518 395
- JP-A- 2008 148 951
- JP-A- 2009 018 176
- JP-A- 2009 537 024
- JP-B2- 3 675 482
- JP-B2- 4 348 338
- US-A- 4 785 815
- US-A- 4 860 743
- US-A- 5 782 824
- US-B1- 6 736 808

## Description

### Technical Field

The present invention relates to the field of treatment and photodynamic therapy for arrhythmia, such as atrial fibrillation, caused as a result of abnormal cellular electrical conduction or hyperexcitability. The present invention also relates to a catheter that performs photodynamic ablation of cardiac muscle tissue via photochemical reaction.

### Background Art

Radio frequency ablation, cryoablation, laser ablation, and the like are currently available means for blocking electric conduction of cardiac muscle tissue, and such means thermally damage tissue, so as to block electric conduction. Such techniques are employed as means for treating irregular heartbeat caused by abnormal electrical signals, which is known as cardiac arrhythmia.

Tachyarrhythmia is a type of arrhythmia that conducts hyperexcitability to normal cardiac muscle tissue or forms an electric excitation reentry circuit in cardiac muscle tissue. In general, cardiac excitation is controlled at a normal rate (i.e., the sinus rhythm) through excitation caused by the sinoatrial node. In the case of tachyarrhythmia, however, the heartbeat is maintained at a faster rate than the sinus rhythm due to hyperexcitability that takes place in part of the cardiac tissue. The term "reentry circuit" refers to a region where normal electric excitation is not conducted but circles because of the presence of a site of impaired conduction in cardiac muscle tissue. The reentry circuit is associated with persistence of tachyarrhythmia, and development and conduction of hyperexcitability cause tachyarrhythmia attacks. For example, atrioventricular nodal reentry tachycardia (AVNRT) attacks are caused by premature atrial contraction, and such arrhythmia is maintained due to the formation of a reentry circuit in the atrioventricular node and part of the atrium. In such a case, part of the reentry circuit may be blocked by catheter ablation or other means as a radical treatment. In addition, an example of tachyarrhythmia cause of attack of which has been found to exist at a specific site and for which radical treatment is provided for preventing attack includes atrial fibrillation (AF).

For example, atrial fibrillation (AF) is a type of cardiac arrhythmia that is caused by irregular atrial excitation, and such arrhythmia causes thrombotic obstruction, such as cerebral infarction. Paroxysmal atrial fibrillation is caused by an aberration in electrical signals from the left atrium (LA) to the pulmonary vein (PV) in cardiac muscle tissue. When atrial fibrillation occurs, the atrioventricular node receives electrical impulses from many sites from the entire atrium, in addition to the sinoatrial node. The atrioventricular node cannot completely process such impulses and, as a consequence, generates irregular, rapid heartbeats. As a result, the blood remains in the atrium, and it increases the risk of the formation of blood clots. Examples of major atrial fibrillation risk factors include age, coronary disease, rheumatic heart disease, elevated blood pressure, diabetes, and thyrotoxicosis.

A large number of patients with arrhythmia have atrial fibrillation, and such patients account for approximately one-third of all arrhythmia patients. At present, the number of patients is deduced to be approximately 0.73 million, and such number is likely to increase with aging. While 1% or less of the entire population has atrial fibrillation at age 60 or younger, several percent of the population aged in their 60s, 5% in their 70s, and 10% or more in their 80s have atrial fibrillation. Drug therapy is conservative therapy that cannot radically cure the disease, and drug therapy is often ineffective in the case of chronic atrial fibrillation. Atrial fibrillation progresses from paroxysmal fibrillation to chronic atrial fibrillation with the elapse of time, and it becomes a major risk factor causing cardiac failure, cerebral infarction, and the like.

As a radical alternative treatment to drug therapy, catheter ablation is available (see JP Patent Publication (Kokai) No. 2004-130095 A, Carlo Pappone et al., Circulation, 2000; 102; 2619-2628, Mathaniel M. Fried et al., Lasers in Surgery and Medicine 28: 197-203, 2001, Kazushi Tanaka et al., Journal of American College of Cardiology, Vol. 38, No. 7, December 2001, 2079-2086, and Walid Saliba et al., Journal of Cardiovascular Electrophysiology, Volume 13, No. 10, October 2002, 957-961). According to the guidelines issued by academic societies in Europe and U.S.A. (ACC/AHA/ESC) in 2008, catheter ablation of atrial fibrillation was officially announced as the second-line therapy, following drug therapy. The current radio frequency catheter ablation comprises pin-point cauterization, with an electrode at the end. When pulmonary vein isolation is performed, it is necessary to deliver radio frequency current many times in order to form a continuous ablation line surrounding the pulmonary vein. It is difficult to form a completely continuous ablation line, and gaps cause reconductance. In addition, it is difficult to control the intratissue temperature with such technique, the temperature within the intramyocardial depth becomes higher than the temperature that is actually set, which causes popping phenomena, and embolism may be induced by char (blood clot) resulting therefrom. Since the temperature in tissue cannot be determined, the ablation depth cannot be controlled, and serious complications such as esophageal perforation and diaphragmatic disorders have been reported.

Accordingly, development of transmural therapeutic techniques that impart little damage to the atrial tissue and tissues in the vicinity thereof and control thermal damage imparted to the atrial tissue has been desired.

In general, photodynamic therapy is employed for cancer treatment or other purposes. Application of photodynamic therapy (PDT; also referred to as "photochemical therapy") to various types of therapy, in addition to endoscopic therapy for early-stage cancer, has been examined (see JP Patent No. 2,961,074, JP Patent Publication (JP Patent Publication (Kokoku) No. H07-53733 B (1995) and the like). photodynamic therapy is a therapeutic technique comprising administering an agent for photodynamic therapy such as a certain type of porphyrin derivative via intravenous injection or other means, allowing the agent for photodynamic therapy to be selectively absorbed and to accumulate at a tissue site at which a lesion such as cancer tissue to be treated is observed, and be irradiated with a ray such as a laser beam to destroy such tissue. This technique makes use of properties of an agent for photodynamic therapy being selectively accumulated in a lesion and being sensitized by light. At present, however, some therapeutic techniques do not involve the use of accumulating properties. This technique is based on a mechanism that an agent for photodynamic therapy integrated into a lesion is excited by light irradiation, energy of the sensitizer is transferred to oxygen that is present in the lesion to generate active, singlet oxygen, and such active oxygen causes cell apoptosis or necrosis in the lesion.

A method for treatment of arrhythmia by photodynamic therapy involving the use of a fat-soluble porphyrin agent for photodynamic therapy and a balloon catheter has been reported (US Patent Application S2002/0095197), although specific conditions and the like of the therapy have not been reported.

Therefore, a transmural treatment method that causes minimal damage to atrial tissues and tissues surrounding the same and prevents thermal damage to atrial tissues has been desired.

An apparatus that performs ablation of cardiac muscle tissue to treat arrhythmia using photodynamic therapy had been reported (WO 2008/066206). Cardiac muscle tissue ablation using photodynamic therapy is not carried out thermally. Active oxygen (singlet oxygen) generated via photochemical reactions among 3 elements (i.e., an agent for photodynamic therapy light, and oxygen) damages cells to cause necrosis in cardiac muscle tissue, and this involves the use of an optical catheter that can be freely operated in the body. It can be said that such technique is substantially free of the risk of causing complications resulting from difficulties in temperature control, which is a problem for catheter ablation at present. However, no satisfactory therapeutic effects could be observed using such apparatus.

US 4,860,743 discloses that cardiac conditions such as blockages in arteries caused by plaque build-up can be treated intravascularly with laser radiation using an optical fiber having a heat conducting end cap on its distal end. A spherical lens within the end cap focuses a small portion of the laser energy directly into the plaque which is in contact with the tip of the end cap and disperses the remaining energy into the end cap for heating it up. The direct laser energy vaporizes part of the plaque so that the end cap can be easily forced through the blockage, and the tissue in contact with the heated end cap is burned away. A diffuser catheter for the ablation of electrical conduction tissue in the heart employs spaced electrodes for detecting the presence of the conduction tissue, and directs laser energy outwardly through the side of the catheter for vaporizing the tissue.

US 5,782,824 discloses a catheter which uses a steerable outer catheter with an inner catheter rotatable and extendable with respect to the outer catheter. The outer catheter may be positioned within a patient's heart and a portion of the outer catheter may provide movement like that of a windshield wiper by manipulation of a spring. By combining the windshield wiper type movement with the extension and rotation of the inner catheter relative to the outer catheter, a large area within a patient's heart may be readily mapped for conduction tissue and be subjected to laser ablation if appropriate. Various configurations provide flexibility in relatively positioning of windows used to apply laser energy and ring electrodes used to sense conduction tissue. Various anchoring techniques are described.

### Summary of the Invention

An object of the present invention is to provide a catheter for the treatment of arrhythmia by blocking abnormal conduction in the cardiac muscle by photodynamic therapy, the therapeutic effects of which can be evaluated.

The present inventors discovered that a target region could be subjected to ablation precisely by photodynamic therapy while using light for irradiation without damaging tissue surrounding the target region. They also discovered that administration of an agent for photodynamic therapy via intravenous injection or other means would lead to distribution of the agent for photodynamic therapy outside cells at the treatment site within a short period of time after administration of the agent for photodynamic therapy, so that treatment could be initiated without waiting for a long time after administration. Thus, they developed an apparatus for photodynamic therapy (International Publication No. WO 2008/066206). The present inventors refer to "ablation by photodynamic therapy" as "photodynamic ablation."

Further, the present inventors discovered that the occurrence or duration of transmission of electric potential between two points may be measured in order to confirm that photodynamic ablation was adequately performed and the target site in cardiac muscle tissue was necrotized. The present inventors discovered that at least two electrodes for electric potential measurement may be provided in the periphery of the light-emitting window for emitting the light beam from a catheter that performs photodynamic ablation, so that the differences in electrical potential between two points sandwiching the target site in cardiac muscle tissue can be measured, whether or not electricity is transmitted to the target site can be determined, and whether or not the target site is necrotized by photodynamic ablation can be determined in the end. This has led to the completion of the present invention.

An aspect of the present invention provides a catheter according to claim 1.

A further aspect of the present invention provides a photodynamic ablation catheter apparatus according to claim 12.

When a therapeutic apparatus utilizing the photodynamic therapy according to the present invention is used, the abnormal electrical conduction site or hyperexcitability site of the cardiac muscle is subjected to photodynamic ablation via photochemical reactions that necrotize tissue cells with active oxygen instead of heat, so as to block the abnormal conduction site of the cardiac muscle. Accordingly, damages imposed on cardiac muscle tissue and tissue in the vicinity thereof can be small. When such technique is used in the vicinity of the pulmonary vein for the purpose of treatment of atrial fibrillation, side effects such as coarctation resulting from destruction of peripheral tissue by heat can be reduced. In particular, targets of the apparatus of the present invention are subjects who have taken the agents for photodynamic therapy. Since the agent for photodynamic therapy is distributed in the extracellular matrix of the myocardial treatment site shortly after administration of the agent for photodynamic therapy, treatment can be initiated shortly after administration of the agent for photodynamic therapy. According to conventional radio frequency catheter ablation techniques for treatment of arrhythmia, it has been impossible to selectively treat the target sites. This is because the target sites are subjected to ablation with heat, and normal tissue in the vicinity of the target sites would be subjected to ablation due to heat conduction. The apparatus of the present invention, however, does not involve the use of heat that can be conducted, and the apparatus implements photodynamic ablation via photochemical reactions involving the use of a light beam capable of limiting the relevant area. This enables limitation of treatment sites. When the apparatus is used for treatment of atrial fibrillation, for example, side effects such as perforation of peripheral tissues (e.g., the esophagus) can be reduced. Pain resulting from fever can also be avoided. In addition, photodynamic ablation can be performed more continuously than is possible with ablation with heat. This can shorten an operation duration.

Further, provision of at least two electrodes for potential measurement in the periphery of the window that emits a light beam enables determination regarding whether or not cardiac muscle tissue cells have been led to necrosis by photodynamic ablation at the target site irradiated with a light beam. This enables evaluation of the effects of photodynamic ablation via photochemical reactions using a catheter.

This description includes part or all of the content as disclosed in the description and/or drawings of Japanese Patent Application No. 2010-042669, which is a priority document of the present application.

### Brief Description of the Drawings

Fig. 1 shows a distal end of a coaxial-light-emission catheter. Fig. 1A shows a front view and Fig. 1B shows a side view.
Fig. 2 is a cross-sectional view of a distal end of a coaxial-light-emission catheter.
Fig. 3 is a side view of a distal end of a lateral-light-emission catheter.
Fig. 4 is a cross-sectional view of a distal end of a lateral-light-emission catheter comprising a mirror. An electrode may serve as a mirror.
Fig. 5 is a cross-sectional view of a distal end of a lateral-light-emission catheter comprising a prism. In Fig. 5, there is an air-containing space surrounded by a light-emitting window 2, an electrode 3, and a prism 6a.
Fig. 6 is a cross-sectional view of a distal end of a lateral-light-emission catheter comprising a GRIN lens.
Fig. 7 shows a configuration of a marker for monitoring the direction of laser beam emission. Fig. 7A to Fig. 7C each show a catheter comprising two markers 7 mounted thereon in a diagonal manner relative to the axial direction of the catheter, Fig. 7D shows a catheter comprising two markers 7 mounted thereon in an approximately vertical manner relative to the axial direction of the catheter, and Fig. 7E shows a catheter comprising two markers 7 mounted thereon in an asymmetric manner when observed from the side.
Fig. 8 shows the whole catheter according to the present invention. Fig. 8A shows an extended catheter end and Fig. 8B shows a bent catheter end. In Fig. 8A, A-A represents a cross-sectional view.
Fig. 9 schematically shows a photodynamic ablation apparatus used for cardiac muscle tissue comprising the catheter of the present invention.
Fig. 10 shows an experimental system for blocking electric conduction in the cardiac muscle by photodynamic therapy using cardiac muscle tissue excised from a rat.
Fig. 11 shows a positional relationship among the electrically-stimulated site, the measuring electrode, and the laser beam irradiated site in an experiment for blocking electric conduction in the cardiac muscle by photodynamic therapy using cardiac muscle tissue excised from a rat.
Fig. 12 shows a waveform of transmitted stimulus at the measuring electrodes A and B measured in the experiment for blocking electric conduction in the cardiac muscle by photodynamic therapy using cardiac muscle tissue excised from a rat. Fig. 12(a) shows a waveform observed before photodynamic therapy and Fig. 12(b) show a waveform observed 5 minutes after photodynamic therapy.
Fig. 13 shows an experimental system for verification of immediate conduction blocking using swine.
Fig. 14 shows the positions of a laser beam irradiated site and an electrode in cardiac muscle tissue in an experiment for verification of immediate conduction blocking using swine.
Fig. 15 shows an electric potential waveform outside the cells at the electrode B before and after laser beam irradiation in an experiment for verification of immediate conduction blocking using swine.
Fig. 16 shows therapeutic effects deduced based on the electric potential waveform at an electrode when it is included in the laser beam emission site.
Fig. 17 shows therapeutic effects deduced based on the electric potential waveform at an electrode when it is not included in the laser beam emission site.

### Embodiments for Carrying out the Invention

Hereafter, the present invention is described in detail.

The catheter that performs photodynamic ablation of cardiac muscle tissue using photodynamic therapy according to the present invention is capable of permanently blocking abnormal electrical conduction in cardiac muscle tissue. For example, tachyarrhythmia and atrial fibrillation are treated by permanently blocking abnormal electrical conduction (electrical entrance) in the tissue.

The term "PDT," which can be used interchangeably with photodynamic therapy or photochemical therapy, used herein refers to a therapeutic method utilizing photochemical reactions that damage and destroy lesions through the presence of a photosensitizer (a PDT agent; an agent for photodynamic therapy) and a light beam capable of exciting an agent for photodynamic therapy.

The catheter of the present invention comprises a light-emission site, and it is capable of emitting a light beam from the light-emission site. The catheter is inserted into the heart through a major vein or artery, an agent for photodynamic therapy is administered to a target site of cardiac muscle tissue, and the target site is irradiated with a laser beam so as to destroy the target tissue. The catheter of the present invention can be used and operated in the blood vessel or cardiac lumen. The blood vessel is preferably of the heart.

The term "catheter" refers to a tubule that can be inserted into a blood vessel. The catheter of the present invention comprises a light transmission means inserted into the tubule or provided in the tubule.

In the present invention, the term "abnormal electrical conduction" in cardiac muscle tissue can be used interchangeably with "reentry," whereby electrical excitation occurring in cardiac muscle is not transmitted unidirectionally but rather turns in the vicinity of (excitation reentry). Reentry is classified as anatomical reentry, which is caused by a specific heart tissue structure, and functional reentry, which can occur at any cardiac muscle tissue site in the heart due to decreased cardiac muscle conduction at a local region and increased nonuniformity of refractory period (time during which, after electrical excitation of cardiac muscle cells occurs once, no response occurs even with an inflow of an electrical stimulus).

An example of the former is atrioventricular nodal reentrant tachycardia (AVNRT) in which reentry occurs when the atrioventricular node has a fast conduction pathway and a slow conduction pathway. Further, reentry caused by occurrence of an accessory conduction pathway between the atrium and the ventricle that is different from the original conduction pathway and passes through the Kent bundle is also a representative form of anatomical reentry, and it causes Wolff-Parkinson-White syndrome (WPW syndrome).

An example of the latter is reentry that causes persistent atrial fibrillation and occurs at any position on the atrium. Furthermore, examples of abnormal electrical excitation include abnormal automaticity and triggered activity. Cardiac muscle cells in atriums and ventricles (working myocardium) naturally have an autonomous excitation function (automaticity), and electrical excitation is usually controlled by the sinoatrial node and the atrioventricular node at superior positions (referred to as "special cardiac muscles"). When resting potential becomes shallow for some reason, automaticity may occur in the working myocardium. This phenomenon is referred to as "abnormal automaticity." The phenomenon of electrical excitation occurring at abnormal timings due to a membrane potential change that occurs during the course of repolarization (after action potential is exhibited, potential settles at original resting potential) of an action potential (potential when the membrane potential of a cardiac muscle cell becomes higher than resting potential due to depolarization) (early after depolarization: EAD) and a membrane potential change that occurs after completion of repolarization (delayed after depolarization: DAD) is referred to as "triggered activity." Such abnormal electrical excitations can cause various arrhythmic conditions. Hyperexcitability from the left atrium to the entrance of the pulmonary vein, which is said to be a major cause of atrial fibrillation, is considered to be either abnormal automaticity or triggered activity, and such excitations are collectively referred to as "focal activity" (local focal excitation).

An abnormal electrical conduction site in the cardiac muscle can be treated by photodynamic ablation using the catheter of the present invention. Treatment of an abnormal electrical conduction site in the cardiac muscle via photodynamic ablation may be occasionally referred to as blocking of abnormal electrical conduction, blocking of an abnormal electrical conduction pathway, blocking of reentry (accessory conduction pathway), or formation of an abnormal electrical conduction block. When the above-mentioned automaticity is formed at a site other than the sinoatrial node and the atrioventricular node, further, the site may be referred to as a site at which hyperexcitability occurs or a site having abnormal automaticity. The site at which hyperexcitability occurs is also a site that generates excess electrical signals in the stimulus transmission system. A site having such a site at which hyperexcitability occurs can be necrotized by photodynamic ablation using the catheter of the present invention. In such a case, abnormal electrical conduction in the cardiac muscle is blocked by necrotizing the site at which hyperexcitability occurs. Accordingly, such a case may also be referred to as blocking of abnormal electrical conduction.

Diseases that can be treated using the catheter of the present invention include arrhythmia caused by the presence of the above-mentioned abnormal electrical conduction site or site at which hyperexcitability occurs, and in particular, tachyarrhythmia. Examples of such tachyarrhythmia include paroxysmal supraventricular tachycardia (PSVT), such as atrioventricular reentrant tachycardia (AVRT; WPW syndrome) and atrioventricular nodal reentrant tachycardia (AVNRT), and atrial flutter, atrial tachycardia, atrial fibrillation (AF) (which are examples of supraventricular tachyarrhythmia), and ventricular tachyarrhythmia, such as ventricular tachycardia.

In the case of atrioventricular reentrant tachycardia, an accessory conduction pathway connecting the ventricle and the atrium exists in addition to the atrioventricular node and the His bundle. Thus, an electrical signal that has been transmitted to the ventricle returns to the atrium. In the case of atrioventricular nodal reentrant tachycardia, no accessory conduction pathway exists. However, a conduction pathway of loop electrical signals is formed with a fast route and a slow route because of differences in the speed of transmission of the electrical signal inside one atrioventricular node. Since the electrical signal continues to circulate in the atrioventricular node and alternately stimulates the atrium and ventricle, tachyarrhythmia develops. Atrial flutter is caused by abnormal electrical activity in which an electrical signal continues to circulate in the right atrium in circle. In the case of atrial tachycardia, a site at which hyperexcitability occurs exists in the atrium. Atrial fibrillation is caused by hyperexcitable conduction in the left atrium-pulmonary vein junction. Ventricular tachycardia is caused by looped abnormal electrical signal transmission that occurs in the vicinity of the cardiac muscle damaged by myocardial infarction or the like.

The application range of ablation is specified by the Japanese Circulation Society (Guidelines for Diagnosis and Treatment of Cardiovascular Diseases, Guidelines for Non-Pharmacotherapy of Cardiac Arrhythmias, Jpn. Circulation J 65 (Suppl. V): 1127, 2001), and a target of the therapy can be selected based on such guidelines.

Accordingly, the site subjected to photodynamic ablation using the catheter of the present invention is the above-mentioned abnormal electrical conduction site or site at which hyperexcitability occurs in the cardiac muscle, which causes arrhythmia. Examples thereof include portions of the cardiac muscle including the atrium such as the atrial septum, the ventricle, the atrial wall, a portion of the ventricular wall and the coronary sinus, a portion of the superior and inferior vena cava, and a portion in the vicinity of the junction of the vein and the cardiac muscle. The site subjected to photodynamic ablation can be suitably determined based on the type of arrhythmia, and an abnormal electrical conduction site or site at which hyperexcitability occurs that causes arrhythmia can be determined by mapping. Thus, the site can be subjected to photodynamic ablation. Photodynamic ablation can be performed in a linear or dotted manner, which can be suitably determined based on the target photodynamic ablation site.

For example, some tissues of the target abnormal left atrium exist in a region in which electrical excitation that causes an attack of atrial fibrillation is conducted to the left atrium. Examples of such region include a portion of the cardiac muscle in the vicinity of the junction between the pulmonary vein (PV) and the left atrium in the heart. The cardiac muscle portion at the junction of the pulmonary vein and the left atrium corresponds to a portion in the vicinity of the entrance of the pulmonary vein. The portion in the vicinity of the junction between the pulmonary vein and the left atrium in the heart is preferable. When tissue in the vicinity of the junction between the pulmonary vein and the cardiac left atrium is destroyed, for example, electrical connection between the left atrium and the pulmonary vein is eliminated (i.e., a conduction block is formed), the pulmonary vein is electrically isolated, excitation is not conducted, and atrial extrasystoles originating in the pulmonary vein, which causes atrial fibrillation, disappear. In such a case, a portion of the junction between the pulmonary vein and the cardiac left atrium may be destroyed, but the entire circumference is preferably treated using the apparatus of the present invention to destroy significant portions of the region in the circumferential direction of the tissue. Alternatively, tissues at the junction of two pulmonary veins (i.e., the superior and inferior pulmonary veins) and the left atrium may be individually destroyed, or two veins may be enclosed and destroyed collectively. Further, tissue at the junction of four pulmonary veins and the left atrium may be enclosed and destroyed collectively. When the pulmonary vein is isolated, tissue is preferably destroyed continuously in a linear manner. The catheter used for photodynamic ablation in photodynamic therapy of the present invention is suitable for continuous photodynamic ablation in a linear manner.

In addition to the isolation of the pulmonary vein, the canopy of the left atrium and the isthmus between mitral annular rings may be destroyed in a linear manner for the treatment of atrial fibrillation.

In the present invention, blocking of electrical conduction from the above-mentioned pulmonary vein to the left atrium may be expressed as "formation of a conduction block between the left atrium and the pulmonary vein" or "performance of photodynamic ablation for electrical pulmonary vein (PV) isolation." The procedure for enclosing and destroying tissue at the junction of four pulmonary veins and the left atrium collectively may be referred to as the Box Isolation technique.

The catheter of the present invention comprises a light beam transmission pathway as a light beam transmission means for transmitting a light beam to a distal end of the catheter. The distal end of the catheter is equipped with a light-transmissive light-emitting window for emitting the light beam transmitted by the light beam transmission means toward the target site. In the present invention, the "light-emitting window" may be occasionally referred to as a "light-emitting site." The catheter of the present invention further comprises at least two electrodes at the distal end.

The thickness of the catheter of the present invention is 5 to 9 Fr and it is preferably 6 to 8 Fr. The light transmission pathway is positioned inside the catheter so as to avoid direct contact with tissue. The light beam that has passed through the light transmission pathway is emitted from the light-emitting window and then the target site is irradiated with the light beam (i.e., cardiac muscle tissue). The end of the catheter of the present invention may have a structure that freely bends. To this end, for example, a tension wire can be provided in the catheter, and the end can be bent by pulling of the tension wire. Furthermore, the end may have been bent beforehand so as to match the shape of a treatment site. A catheter that is generally used as a heart catheter can be used. The catheter of the present invention may comprise a guide sheath or a guide wire for achieving the insertion of a catheter into the target site.

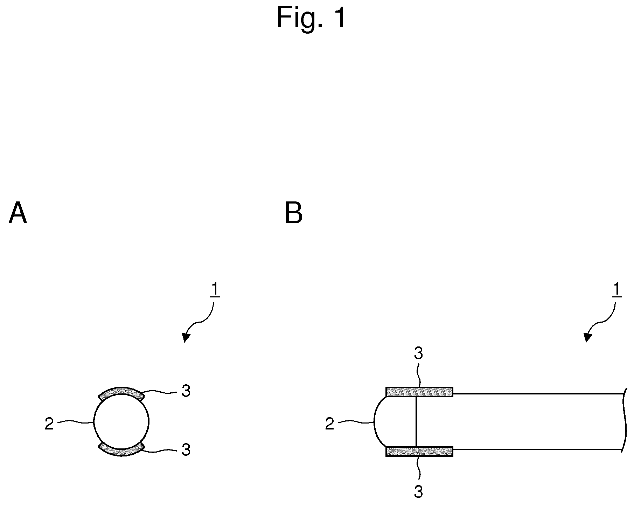

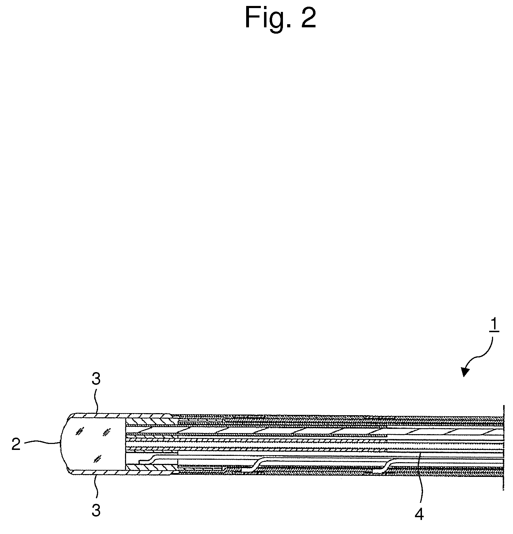

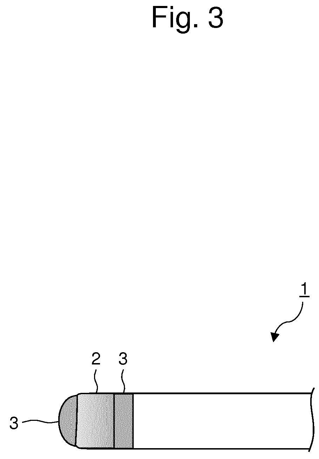

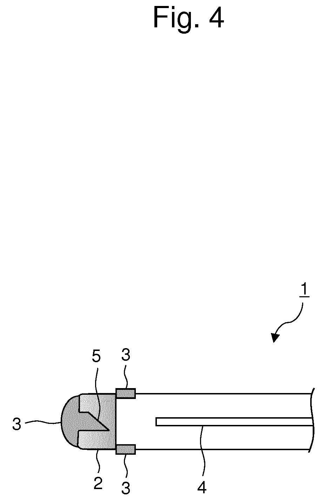

The light beam emitted from the end of the light beam transmission pathway is emitted to the outside of the catheter through the light-emitting window and the target site is irradiated. The light-emitting window is provided in the vicinity of the distal end of the catheter. A region "in the vicinity of the distal end" is a region near the end located on the opposite side of the end connected to the light beam generator (the proximal end), and such expression refers to the distal end and the region within about several tens centimeters from the distal end. For example, the light-emitting window is provided at the end of the catheter or a side in the vicinity of a distal end of the catheter. When the light-emitting window is provided at an end of the catheter, for example, light is emitted coaxially with the catheter. When the light-emitting window is provided on a side in the vicinity of a distal end of the catheter, light is emitted laterally relative to the axial direction of the catheter. When light is emitted laterally, the direction (angle) of emission is not limited. Light may be emitted in a vertical direction or in a diagonally forward direction relative to the long axial direction of the catheter. When light is emitted at an angle of 0° to 90° relative to the long axial direction of the catheter designated as the standard (0), in general, such emission is referred to as "lateral light emission." The direction of light emission refers to the direction of the central axis of a laser beam (i.e., the direction of the luminous flux). The former type is referred to as a coaxial-light-emission catheter and the latter type is referred to as a lateral-light-emissioncatheter. The catheter of the present invention may be capable of coaxial and lateral light emission. Light-emitting windows may be provided both at the end of the catheter and a region in the vicinity of the distal end. The light-emitting window is composed of a light-transmissive material. Examples of such materials include glass, such as quartz glass, sapphire glass, and BK7 (borosilicate crown optical glass), and transparent resins. Such light-emitting window may be occasionally referred to as an "optical window" in the present invention. The light-emitting window may have functions of condensing light emitted from the end of a laser transmission pathway, scattering the light, or changing the direction of emission. The configuration of the light-emitting window is not limited, provided that the light beam emitted through the light beam transmission pathway inside the catheter is transmitted therethrough and emitted to the outside of the catheter. For example, the light-emitting window can be in the form of a plate, lens, cube, or cylinder (column). Specifically, the light-emitting window is made of an optical lens such as a concave or convex lens, a gradient index lens such as a GRIN lens, an optical element such as a prism or mirror, or a liquid material, for example. The surface of the light-emitting window is made from a material or has a structure that suppresses interfacial reflection of substances having different refractive indexes. An example of a substance that suppresses interfacial reflection is an index-matching material. Specific examples include antireflection coatings, such as matching oil and AR coating. An example of a structure that suppresses reflection is a nanostructure that is smaller than an optical wavelength. Specific examples of light-emitting windows include the light-emitting windows of the catheters exemplified in Fig. 1 and Fig. 3. The light-emitting window of the catheter shown in Fig. 1 is in a cylindrical form with a spherical end. The catheter shown in Fig. 1 is a coaxial-light-emission catheter. Fig. 1B shows a side view when the catheter end is designated as the front, and Fig. 1A shows a front view. Fig. 2 is a cross-sectional view. The catheter shown in Fig. 3 is a lateral-light-emission catheter, which has a ring-shaped light-emitting window. Fig. 4 shows a cross-sectional view of a distal end of the catheter having a ring-shaped light-emitting window. A plate-like light-emitting window can also be used. Such plate-like light-emitting window may have a certain degree of curvature relative to a curve of the catheter. When a plate-like light-emitting window with a certain degree of curvature surrounds the entire catheter, the light-emitting window has a ring shape.

The catheter of the present invention comprises at least two electrodes. Such electrodes function at least as electrodes for potential measurement. The catheter of the present invention may comprise a conducting electrode. The electrode for potential measurement may also serve as a conducting electrode. Alternatively, a conducting electrode used only for conduction may be provided separately from an electrode for potential measurement used only for potential measurement. The catheter of the present invention is required to comprise at least two electrodes for potential measurement.

The conducting electrode is capable of passing an electric current to a site with which the electrode is in contact. Such conducting electrode is connected to a lead wire provided inside the catheter, and it is then connected to a power unit through the lead wire. The conducting electrode can be used for conducting electricity to the target site to heat and necrotize such site. The catheter of the present invention performs photodynamic ablation of cardiac muscle tissue via photochemical reactions, and ablation can also be carried out with heating with the supplemental use of a conducting electrode.

An electrode for potential measurement can be used for measuring potential at a target site with which the electrode is in contact. There are at least two electrodes for potential measurement, and these electrodes are located in such a manner that they are capable of measuring potential at sites on both sides of cardiac muscle tissue that has been subjected to ablation with the light beam emitted from the light-emitting window, and potential differences between such sites. Specifically, at least two electrodes for potential measurement are provided in the periphery of the light-emitting window so as to sandwich the same. The electrodes may be provided in the periphery of the light-emitting window while they are in contact with the light-emitting window. Alternatively, the electrodes may be located within a short distance from the light-emitting window without being in direct contact therewith. For example, the electrodes may be provided within 5 mm and preferably within 1 mm from the light-emitting window. Also, the electrodes sandwich the light-emitting window in such a manner that the light-emitting window is located on a line connecting an electrode to another electrode among a plurality of electrodes for potential measurement. The light-emitting window may be provided with a window frame used for mounting the light-emitting window on the catheter. When a window frame is provided, electrodes for potential measurement may be provided on the window frame. Also, an electrode for potential measurement may serve as a window frame. The term "at least two electrodes" mentioned above refers to, for example, 2, 3, 4, 5, or more electrodes, and 2 electrodes are preferable. Alternatively, electrodes for potential measurement may be provided in regions other than the periphery of the light-emitting window. For example, a plurality of electrodes may be provided outside the distal end of the catheter. In such a case, for example, ring-shaped electrodes surrounding the periphery of the catheter can be used. When a plurality of such ring-shaped electrodes are provided, the distances between ring-shaped electrodes are approximately within 10 mm, and preferably within 5 mm. Also, the electrode configurations are not limited to those mentioned above, and an electrode may be in the form of a line, plane, ribbon, cylinder, dome, or the like. A plane form may be a flat or curved plane. For example, a coaxial-light-emission catheter shown in Fig. 1 has two planar electrodes on the outer circumference of the catheter end. In this case, such two electrodes are referred to as the first electrode and the second electrode. A lateral-light-emission catheter shown in Fig. 3 has a round-nose cylinder-shaped (dome-shaped) electrode located preferably in the vicinity of a distal end, and more preferably at a site closer to the distal end than the light-emitting window, and a ring-shaped electrode at a site closer to the proximal end than the light-emitting window. In such a case, an electrode provided preferably in the vicinity of the distal end, and more preferably at the distal end, is referred to as the first electrode, and a ring-shaped electrode provided at a proximal site is referred to as the second electrode. The electrode material may be an SUS material. Use of materials that would not adversely affect organisms is preferable. Examples thereof include gold, silver, platinum, tungsten, and palladium, alloy of any thereof, and Ni-Ti alloys, and titanium platinum.

The electrodes for potential measurement can be connected to a potential measuring instrument through a lead wire and used to measure potential. In such a case, two counter electrodes may be used and potential differences between two electrodes may be measured. Alternatively, a reference electrode is separately provided in another site, such as the surface of the outer skin of a subject, and potential relative to the reference electrode may be measured. With the use of at least two electrodes for potential measurement, potential differences between two points of cardiac muscle tissue that sandwich the site subjected to photodynamic ablation via photochemical reactions can be measured.

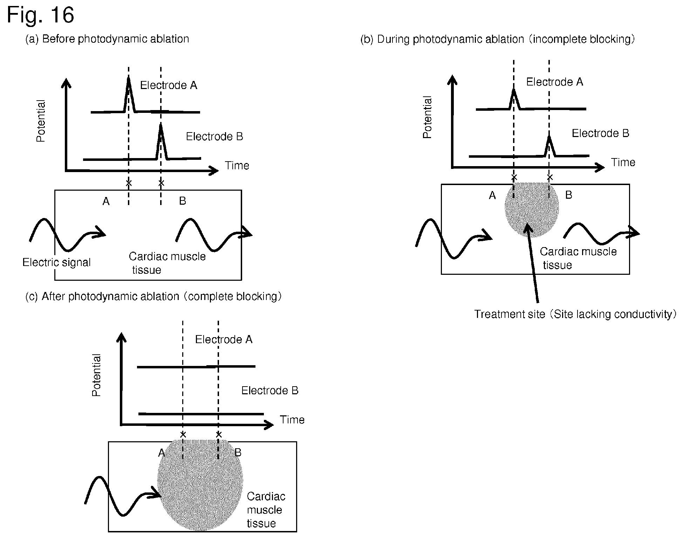

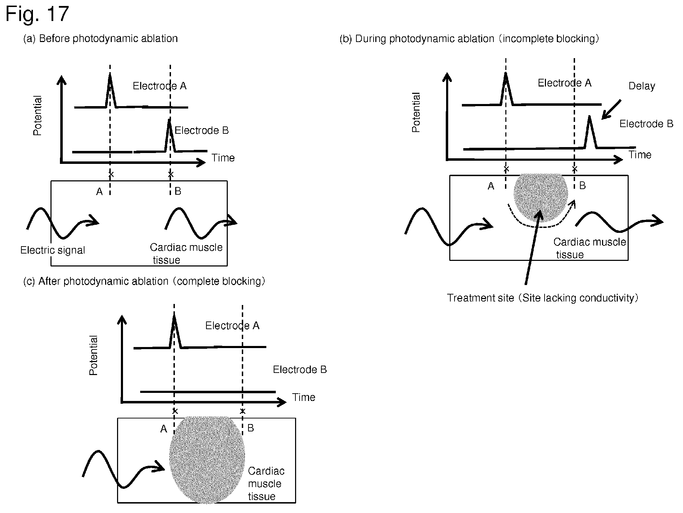

When cardiac muscle tissue is necrotized by photodynamic ablation, electrical conduction becomes difficult or impossible at the necrotized site. With reference to Fig. 16 and Fig. 17, changes in the electric conductivity of cardiac muscle tissue deduced based on electric potential waveforms at the electrodes are described in two ways; that is, a case in which the potential measuring site includes the photodynamic ablation site and a case in which it does not include the photodynamic ablation site. A case in which two electrodes are used is considered herein.

A case in which an electrode is included in a light emission site is first described. When there are two electrodes A and B, electrical signals transmitted at given speeds are first measured at electrode A and then at electrode B. Electrical signals are transmitted in cardiac muscle tissue in the manner as shown in Fig. 16(a) prior to photodynamic ablation. When electric conductivity begins to disappear because of photodynamic ablation in the vicinity of the electrodes, amplitudes in the electric potential waveforms observed at electrode A and electrode B decrease, as shown in Fig. 16(b). Since electrical signals are not transmitted if electric conductivity is completely lost in cardiac muscle tissue as shown in Fig. 16(c), amplitudes in electric potential waveforms also disappear. A case in which an electrode is not included in a light emission site is described next. Electrical signals are transmitted in cardiac muscle tissue in the manner as shown in Fig. 17(a) prior to photodynamic ablation. When electric conductivity begins to disappear because of photodynamic ablation in a region between two electrodes, electrical signals are transmitted while bypassing the site at which electric conductivity has disappeared as shown in Fig. 17(b). This generates a temporal difference (delay) in the electric potential waveform at electrode B, compared with that prior to photodynamic ablation. Since electrical signals are not transmitted if electric conductivity is lost across all the cardiac muscle tissue wall layers as shown in Fig. 17(c), electrical signals are not detected at electrode B.

Thus, potential may be measured using at least two electrodes for potential measurement, so that photodynamic ablation effects at a site subjected to photodynamic ablation via photochemical reactions can be evaluated. When three or more electrodes for potential measurement are provided, amplitudes in the electric potential waveforms or delays in electric transmission among any of three or more points can be measured, and photodynamic ablation effects can be more precisely evaluated.

When photodynamic ablation is performed via photochemical reactions, it is preferable that the catheter end be bent at the time of light beam emission, so that the target site is irradiated with the light beam. When the catheter end is brought into contact with cardiac muscle tissue upon bending, changes occur in the potential measured with electrodes for potential measurement. This enables the judgment regarding the occurrence of contact. Judgment regarding the occurrence of contact also enables judgment regarding whether or not the catheter end is positioned in the correct direction relative to the target site.

It is preferable that a light transmission means provided inside the catheter be an optical fiber with light conductivity of 90% or higher. A quartz optical fiber or plastic fiber is preferably used. An optical fiber is provided inside the catheter, and at least one optical fiber is used. When a plurality of optical fibers are used, a plurality of optical fibers exerting the same functions, optical fibers used for excitation light of photosensitive pigments and optical fibers for receiving the light returned therefrom or fluorescence, optical fibers for coaxially emitting light beam, optical fibers for laterally emitting light beam, and optical fibers for diagonally emitting light beam, or optical fibers for transmitting light beams with different wavelengths, are used. The outer diameter of an optical fiber used is 100 µm to 400 µm, and preferably 200 µm to 300 µm. The core diameter of an optical fiber used is 50 µm to 300 µm, and preferably 100 µm to 200 µm. The numerical aperture (NA) of an optical fiber is 0.1 to 1, and preferably 0.2 to 0.5. The diameter of laser beam emission on the window surface at the catheter end is 0.2 mm to 3 mm, and preferably 1 mm to 2.5 mm. When cardiac muscle tissue, blood, physiological saline, high-molecular-weight dextran, or contrast medium is used as a medium through which the light emitted from the light-emitting window passes toward the target site, the laser beam emission solid angle is 3° to 60°, and preferably 5° to 45° at the catheter end. An end of an optical fiber is connected with a light beam generator, and an optical fiber is capable of transmitting the light beam generated by the light beam generator to the target site.

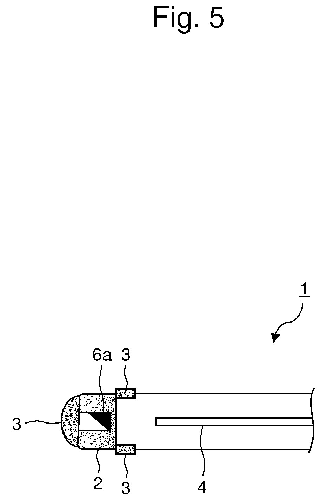

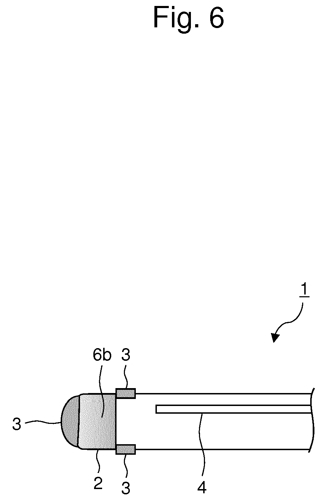

An optical fiber may or may not be in contact with the light-emitting window. When the light-emitting window is provided at the catheter end and the light beam transmitted through an optical fiber is emitted through the light-emitting window composed of a lens along the axial direction of the catheter or the transmitted light beam is emitted through the light-emitting window composed of a prism in a lateral direction instead of a long axial direction of the catheter, for example, an optical fiber may be brought into contact with the light-emitting window to reduce light loss resulting from diffusion or reflection. When an optical fiber is in contact with the light-emitting window, the contact surfaces are a plane surface and a plane surface, a curved surface and a curved surface, a plane surface and a curved surface, or a convex surface and a concave surface. The contact surfaces of the optical fiber and the light-emitting window are made of substances or have structures that suppress the interface reflection of substances having different refractive indexes. A specific example of a substance that suppresses reflection is an index-matching material. More specifically, such substance can be an antireflection coating, such as matching oil or AR coating. An example of a structure that suppresses reflection is a nanostructure that is smaller than an optical wavelength. When the light beam transmitted through an optical fiber provided inside the catheter is emitted laterally instead of along the long axial direction of the catheter using a mirror, the light beam emitted from the optical fiber reflects off the mirror, and the target site is then irradiated through the light-emitting window. Accordingly, the optical fiber is not necessarily in contact with the light-emitting window. When the light is emitted laterally, as shown in Fig. 4, a mirror may be fixed at a given angle inside the catheter at its distal end, so that the light beam emitted from the end of the optical fiber reflects off the mirror and is then emitted to the outside through the light-emitting window. In such a case, an electrode may be integrated with a mirror; that is, an electrode may serve as a mirror. The angle for mounting a mirror may be adjusted, so that a light beam can be laterally emitted at an arbitrary angle. While the catheter shown in Fig. 4 uses a mirror, it is sufficient for the catheter to contain inside itself a construct such as an optical component or element capable of reflecting a light in an arbitrary direction. For example, a prism may be used instead of a mirror (Fig. 5), or a mirror may be provided on a light-reflecting surface of a prism. Also, a gradient index lens such as a GRIN lens may be used (Fig. 6) to emit a light beam in a lateral direction. Further, a mirror, a prism, and a lens may be used in combination. Use of such components in combination enables the light beam emission direction to be changed in two steps, for example. In such a case, the same components may be used in combination, or different components, such as a mirror and a prism, a mirror and a lens, a lens and a prism, or a mirror, a prism, and a lens, may be used in combination. In order to reduce the influence at the interface on the optical pathway along which the light beam emitted from the end of an optical fiber reflects and reaches the light-emitting window located on the side, the light-emitting window may be in the form of a container, a mirror may be provided in such container, and a liquid material, such as a solution, may be introduced thereinto. Further, the light-emitting window may be a transparent solid comprising a mirror embedded therein. In such a case, a window may be prepared from transparent resin, and a mirror for reflecting a light beam may be embedded therein at the time of preparation. Furthermore, the internal structure of the catheter may be designed so as to reflect light, and an optical fiber may be processed (e.g., diagonal sanding) to form a refractive surface from which a light beam reflects.

Hereafter, the catheter of the present invention is described with reference to Figs. 1 to 6. It should be noted that the catheter described herein is merely an example and the catheter of the present invention is not limited to the catheters shown in Figs. 1 to 6.

The catheter 1 shown in Figs. 1 and 2 comprises an optical fiber 4 inside itself and a light-emitting window 2 made of a cylindrical optical element at the end thereof. The catheter 1 shown in Figs. 1 and 2 emits a light beam along the long axis thereof. The optical fiber 4 is in contact with the light-emitting window 2, a light beam transmitted through the optical fiber 4 passes through the light-emitting window 2 while suppressing loss caused by reflection or scattering, and the light beam is then emitted to the outside. The periphery of the light-emitting window 2 is covered with a window frame. The window frame is connected to the catheter, and the light-emitting window 2 is fixed to the catheter 1 with the aid of the frame. When the light beam emitted through the optical fiber 4 passes through the inside of the optical window 2, the light beam is partially applied to the window frame that covers the side of the light-emitting window 2. If the light beam is absorbed by the window frame, the efficiency of light beam irradiation of the target site becomes lowered. Accordingly, it is preferable that the window frame covering the light-emitting window 2 be made of a material that reflects light beams or the inner surface thereof be coated with a material that reflects light beams. Use of a material having 90% or higher optical reflectivity of light at a wavelength of 600 nm to 700 nm is preferable. Examples of such materials include gold, silver, aluminum, copper, and dielectric substances. The optical reflectivity of gold, silver, aluminum, and copper of light having a wavelength of 700 nm is 95.5%, 98.3%, 90.3%, and 96.6%, respectively. Coating may be provided by forming a thin film of such metal on a surface by means of plating, sputtering, vapor deposition, or other means. The inner surface of the window frame may be treated to be glossy, so that the light undergoes specular reflection. Alternatively, surface roughness may be retained, so that the light undergoes diffuse reflection. It is preferable that the inner surface of the window frame have a specular finish. When a lateral-light-emission catheter allows a light beam to reflect off a mirror, it is preferable that a mirror surface be coated with any of the materials having high reflectivity mentioned above.

Further, the window frame is provided with at least two electrodes 3 for potential measurement. As shown in Fig. 1A, for example, the electrodes for potential measurement 3 may be provided at both sides of the light-emitting window 2 opposite to each other in such a manner that the two electrodes 3 partially cover the exterior of the window frame. Since the electrodes 3 for potential measurement are used for measuring the potential in the vicinity of the target site of cardiac muscle tissue, it is necessary to bring the electrodes into contact with an area in the vicinity of the target site of cardiac muscle tissue. To this end, the electrodes are provided at the end of the catheter. The electrode 3 is connected to a lead wire, the electrode 3 is then connected to a potential measuring instrument through the lead wire, and potential at the target site into contact with which the electrodes 3 for potential measurement had been brought can then be measured.

The catheter shown in Figs. 3 to 6 is a lateral-light-emission catheter comprising an optical fiber 4 inside itself and a light-emitting window 2 made of an optical element on the lateral side of the catheter distal end. In the case of the catheter shown in Fig. 4, the light beam emitted through an optical fiber reflects off the mirror 5 and is emitted to the outside through the light-emitting window 2. In the case of the catheter shown in Fig. 5, the light beam emitted through an optical fiber is deflected by a prism 6a, and it is emitted to the outside through the light-emitting window. In the case of the catheter shown in Fig. 6, the light beam emitted through an optical fiber is deflected by a GRIN lens 6b, and it is emitted to the outside through the light-emitting window 2. At least two electrodes 3 for potential measurement can be provided in the periphery of the light-emitting window 2 so as to sandwich the window in the case of such lateral-light-emission catheter, and potentials at two points of cardiac muscle tissue can then be measured. The lateral-light-emission catheter shown in Fig. 3 comprises a dome-shaped electrode 3 at its end and a ring-shaped electrode 3 on the opposite side of the light-emitting window.

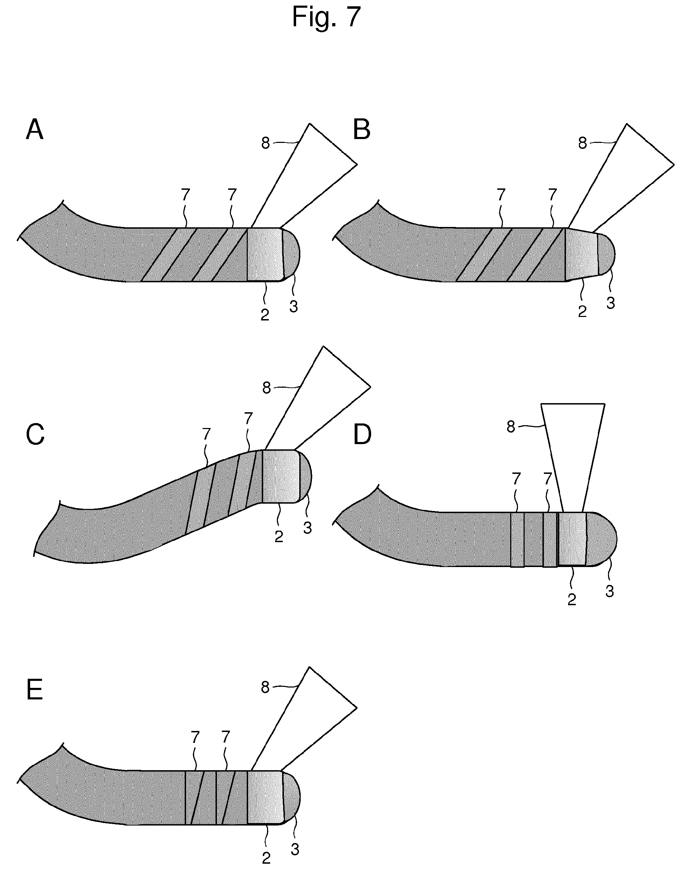

The catheter of the present invention may further comprise a means for monitoring the direction of light beam emission from outside of a body. Such means is capable of external detection when a catheter is inserted into the body. An example of such means is a radiopaque marker mounted at a site in the vicinity of the distal end of the catheter, so that the direction of a light beam being emitted; i.e., the position of the light-emitting window, can be detected. Preferably, the marker is in the form of a line, plane, ribbon, or ring, and it is mounted along the outer circumference of the distal end of the catheter. In such a case, the marker may be provided to intersect the long axis of the catheter. The marker may be preferably mounted on the catheter in a diagonal manner at a given angle relative to the long axial direction of the catheter. Alternatively, a marker may be mounted in an asymmetric manner when a marker mounted in an area in the vicinity of the distal end of the catheter is observed from the lateral direction. Fig. 7 shows an example of a marker mounted on the catheter at its distal end. Fig. 7A to Fig. 7C each show a catheter comprising two markers 7 mounted thereon in a diagonal manner relative to the axial direction of the catheter, Fig. 7D shows a catheter comprising two markers 7 mounted thereon in an approximately vertical manner relative to the axial direction of the catheter, and Fig. 7E shows a catheter comprising two markers 7 mounted thereon in an asymmetric manner when observed along the lateral direction. The configuration of the marker 7 is associated with the position of the window through which the light is emitted from the catheter, and the direction of light beam emission can be detected by observing the marker. For example, the catheter shown in Fig. 7A to Fig. 7C is a lateral-light-emission catheter comprising two markers 7 mounted thereon in a diagonal manner relative to the long axial direction of the catheter. Such catheter emits the light beam 8 in a diagonally forward direction, and the light beam 8 is emitted in a linear direction of the marker 7 mounted in a diagonal manner on the catheter. The catheter shown in Fig. 7D is a lateral-light-emission catheter, which comprises two markers 7 in an approximately vertical manner relative to a long axial direction of the catheter. Such catheter emits the light beam 8 in a long axial direction of the marker 7. In Fig. 7E, two markers 7 are asymmetric when observed from the lateral side, and the thickness of a marker 7 differs from that of the other marker 7 along the long axis of the catheter. The light beam 8 is emitted laterally in the direction in which the marker 7 becomes thickened. When a marker that monitors the direction of light beam emission is mounted, the direction of emission can be targeted to the site of treatment. An X-ray impermeable metal can be used as a radiopaque marker, and platinum, gold, iridium, and an alloy of such metals used in combination are preferable from the viewpoint of biocompatibility. When the apparatus of the present invention comprises a catheter, for example, at least one radiopaque marker (e.g., 2, 3, or more) may be provided at a distal end of the catheter. Also, a marker may serve as an electrode for potential measurement. When a plurality of markers are provided, at least one marker functions as an electrode for potential measurement.

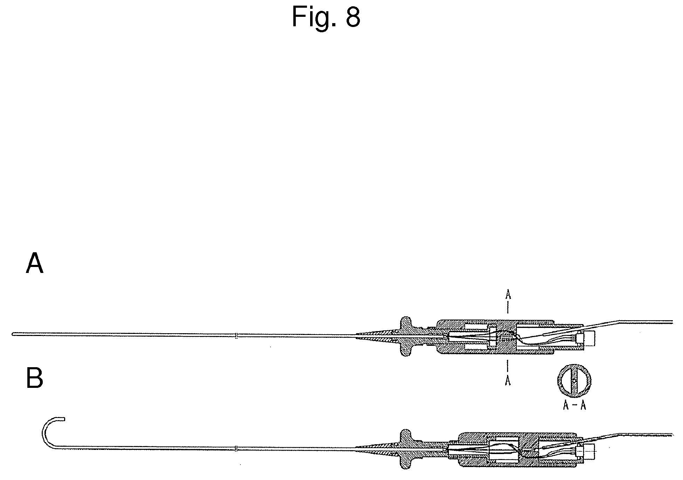

Fig. 8 shows an overall view of the catheter of the present invention.

The catheter of the present invention is inserted into the heart through blood vessels, and it can be used in the cardiac lumen in the presence of blood. In such a case, the light beam emitted from the catheter passes through the inside of the blood for a short distance and reaches the target site. In such a case, physiological saline, polymeric dextran, a contrast medium, a liquid containing artificial erythrocytes, or the like is injected into the cardiac blood vessels through the catheter end, and the light beam may be emitted using such liquid as a medium.

When photodynamic therapy is performed, administration of a sensitizer (e.g., an agent for photodynamic therapy or photosensitive pigment) is necessary. Agents for photodynamic therapy that are to be used in combination with the apparatus of the present invention are not limited, and a known agent for photodynamic therapy can be used in combination with a light beam at an absorption wavelength thereof. Adequate agents for photodynamic therapy and light beam types may be selected. Any agent for photodynamic therapy can be selected from agents for photodynamic therapy having an absorption wavelength at about 630 nm to those having longer absorption wavelengths. For the treatment of arrhythmia, use of an agent for photodynamic therapy that can be easily eliminated from cardiac muscle cells is preferable. Since it is desirable to perform light beam irradiation before an agent for photodynamic therapy is taken up into cells, use of an agent for photodynamic therapy that can remain in the extracellular matrix for a long period of time is preferable. Thus, water-soluble agents for photodynamic therapy are suitable for the treatment of arrhythmia. Examples of such agents for photodynamic therapy include chlorine-based agents for photodynamic therapy having chlorine skeletons, such as ATX-S10 (670 nm) (iminochlorin aspartic acid derivative, Oriental Menthol Industry Ltd., rights were transferred to Photochemical Co., Ltd. in 2000, JP Patent Publication (Kokai) No. 6-80671 A (1996)), NPe6 (664 nm) (talaporfin sodium, Laserphyrin®, mono-L-aspartyl chlorine 6, JP Patent No. 2961074), mTHPC (652 nm), SnET2 (660 nm) (tin etiopurpurin, Miravant Medical Technologies), AlPcS (675 nm) (chloroaluminium sulphonated phthalocyanine), BPD-MA (690 nm) (benzoporphyrin derivative monoacid ring A, QLT Inc.), and Lu-tex (732 nm) (Lutetium Texaphyrin), with talaporfin sodium being particularly preferable. The agent for photodynamic therapy is dissolved in an appropriate buffer such as a phosphate-buffered saline solution, pharmaceutically acceptable additives are added thereto according to need, and the resulting mixture is administered. Examples of additives include solubilizers, such as organic solvents, pH modifiers, such as acids and nucleotides, stabilizers, such as ascorbic acids, excipients, such as glucose, and isotonizing agents for photodynamic therapy, such as sodium chloride.

An agent for photodynamic therapy for performing photodynamic therapy is preferably administered by intravenous injection to a subject to be treated in advance. The agent for photodynamic therapy may be administered by supplying a highly-concentrated agent for photodynamic therapy through a catheter placed in a specific blood vessel, such as the coronary artery to the cardiac muscle. In this case, the apparatus of the present invention comprises a means for supplying an agent for photodynamic therapy (i.e., an apparatus for supplying an agent for photodynamic therapy). The means for supplying an agent for photodynamic therapy comprises, for example, a means for pooling the agent for photodynamic therapy, a means for delivering the agent for photodynamic therapy to a target site, and a means for administering the agent for photodynamic therapy to the target site. Thus, administration of the agent for photodynamic therapy leads the agent for photodynamic therapy to be present at the target site, and the irradiation the target site with a light beam can damage the abnormal electrical conduction site or site at which hyperexcitability occurs by necrosis or the like.

Doses of the agent for photodynamic therapy are not limited. For example, several µl to several ml, and preferably 1 ml to 10 ml of the agent for photodynamic therapy adjusted to several µg/ml to several mg/ml, and preferably 10 mg/ml to 100 mg/ml is administered by intravenous injection. The dose per body weight is 0.1 mg/kg to 10 mg/kg, and preferably 0.5 mg/kg to 5 mg/kg. Further, the agent for photodynamic therapy may be directly administered to the target site by injection or other means.

Light beam irradiation can be initiated immediately or shortly after administration of an agent for photodynamic therapy. For example, the agent for photodynamic therapy is uniformly distributed at a treatment site within 0.5 to 10 hours after administration, preferably within 0.5 to 6 hours after administration, more preferably within 0.5 to 5 hours after administration, and further preferably within 0.5 to 3 hours after administration, and light beam irradiation can then be initiated. At such time, whether an agent for photodynamic therapy suitable for treatment has accumulated at the treatment site can be determined using the concentration of the agent for photodynamic therapy in the blood as an indicator. When a dose of 1 mg/kg is administered to a human, for example, treatment may be carried out by irradiation with a light beam when the concentration is 5 µg/ml to 50 µg/ml, preferably 10 µg/ml to 30 µg/ml, and more preferably 15 µg/ml to 25 µg/ml in the blood plasma. According to the present invention, photodynamic ablation therapy via photodynamic therapy can be initiated shortly after administration of the agent for photodynamic therapy.

When photodynamic ablation therapy via photodynamic therapy is performed on a human, the dose of the agent for photodynamic therapy and the duration from administration of the agent for photodynamic therapy to light beam irradiation can be determined based on conditions determined using animals such as swine, rats, or mice.

In photodynamic therapy comprising administration of an agent for photodynamic therapy and light beam irradiation, cells are damaged by active oxygen. In photodynamic therapy, heat is not generated, and localized treatment is enabled. Accordingly, heat denaturation of proteins does not occur, a target site and tissues surrounding the target site are not necrotized, and a target site can be selectively and assuredly damaged. When only a light beam such as a laser beam is used without the use of an agent for photodynamic therapy, heat can be generated at a site irradiated with a laser beam. Accordingly, surrounding tissues can also be damaged. Thus, the method and the apparatus using photodynamic therapy of the present invention also have excellent effects, compared with a method or an apparatus involving irradiation with a laser alone without the use of an agent for photodynamic therapy.

Types of light beams applied for treatment using the apparatus of the present invention are not limited, and continuous light rays can be used. A wavelength to be irradiated is 600 nm to 800 nm, and a light beam with a wavelength close to the absorption wavelength of an agent for photodynamic therapy to be used can be employed.

Light beams used in the apparatus of the present invention are preferably a continuous laser beam and a semiconductor laser beam. Light emitted from a light emitting diode (LED) light source can also be used (LED light).

When talaporfin sodium is used as an agent for photodynamic therapy, a semiconductor laser beam with a wavelength of 650 nm to 690 nm, preferably with a wavelength of 660 nm to 680 nm, and more preferably with a wavelength of 664 nm ± 2 nm is used. When an LED light-emitting source is used, a red LED with a wavelength of approximately 660 nm is preferable.

The intensity of the light beam to be used for the irradiation refers to the peak intensity, and the relevant unit is W/cm². When photodynamic therapy is performed with light beam irradiation, the total energy density (irradiation dose, J/cm²) also determines the success or failure of the photodynamic therapy, and the intensity or the total energy density can be suitably determined in accordance with the size of the abnormal area to be treated and other conditions. As the intensity of a light beam to be used for the irradiation, ranges of high intensity and low intensity are not limited, and such ranges can be suitably determined in accordance with the type of light beam, the depth of the abnormal site to be treated, and other conditions. For example, the intensity of a light beam can be 1 mW/cm² to 100 W/cm², preferably 1 W/cm² to 50 W/cm², and more preferably 2 W/cm² to 30 W/cm². The duration of irradiation is 10 to 1,000 seconds, preferably 50 to 500 seconds, and more preferably 50 to 200 seconds. Examples of the total energy density on the surface of the site irradiated with the light beam include 1 to 10,000 J/cm², preferably 10 to 2,000 J/cm², more preferably 50 to 2,000 J/cm², and still more preferably 100 to 1,000 J/cm². When blood in cardiac muscle tissue is replaced with a liquid containing artificial erythrocytes, the light absorption coefficient can be reduced. In such a case, 10 to 500 J/cm² is preferable.

Cardiac muscle tissue in a site to a depth of 3 mm to 5 mm from a position irradiated with light can be a target of photodynamic ablation.

When photodynamic ablation therapy is performed on humans, the conditions for light beam irradiation can be determined based on the conditions determined using animals such as swine, rats, and mice.

In a method of necrotizing a target site with heat, tissues surrounding the target site can also be damaged due to heat conduction. In contrast, the method using the catheter of the present invention does not involve the use of heat that can be conducted, but rather it involves the use of a light beam with a reachable region that can be controlled. This enables local treatment. For example, local treatment can be performed without damaging surrounding normal tissues, even when a region at an abnormal electrical conduction site or site at which hyperexcitability occurs in the cardiac muscle is small. In therapy using the catheter of the present invention, temperature increase in a target site following light beam irradiation is within 20°C, preferably within 10°C, and more preferably within 5°C, and the maximum temperature is 60°C or lower, preferably 50°C or lower, and more preferably 45°C or lower.

When a light beam is emitted, it is partially absorbed by a catheter end, and temperature at a region close to a distal end of the catheter can increase. Since the catheter of the present invention involves the use of a material with a high degree of light beam reflectivity inside itself, temperature increase at a region close to a distal end of the catheter can be regulated within 10°C in a blood vessel. Thus, tissue would not be damaged by temperature increase.

### Apparatus comprising the catheter of the present invention

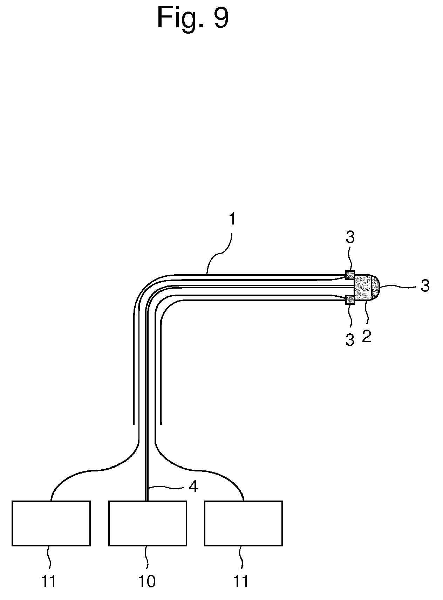

The present invention includes an apparatus for blocking abnormal electrical conduction in the cardiac muscle, an apparatus for treating arrhythmia, or an apparatus for treating atrial fibrillation using photodynamic therapy, which comprises the catheter that performs photodynamic ablation of cardiac muscle tissue via photochemical reactions of the present invention. Such apparatus comprises at least a light transmission means, a catheter comprising a light-emitting window and at least two electrodes for potential measurement in the periphery of the window, and a light beam generation means (i.e., a light beam generator). The catheter may further comprise a conducting electrode and a single or a plurality of electrodes for potential measurement at sites other than those in the periphery of the light beam-emitting window. In addition, the apparatus of the present invention may be equipped with a potential measuring instrument connected to the electrode for potential measurement through a lead wire and a power source connected to the conducting electrode through a lead wire. An example of the apparatus is shown in Fig. 9. A light beam generated by the light beam generator 10 reaches a distal end of the catheter 1 through the optical fiber 4, and it is emitted from the light-emitting window 2. The electrodes for potential measurement that measure potential in the vicinity of the site irradiated with light with the two electrodes 3 for potential measurement are connected to the potential measuring instrument 11 through lead wires. In addition to the means described above, the apparatus of the present invention may comprise a means capable of monitoring the amount of the agent for photodynamic therapy accumulated at the abnormal electrical conduction site or site at which hyperexcitability occurs and the oxygen concentration at the abnormal site for determination of conditions for light beam application. The apparatus may further comprise a means for supplying the agent for photodynamic therapy to the abnormal electrical conduction site or site at which hyperexcitability occurs. Since the apparatus of the present invention does not have a balloon but rather has only a diffusion fiber or a bare fiber, a narrow site or a complex site that cannot be treated with an apparatus having a balloon can be treated.

A light beam generation means can be the light beam generator capable of a generating a light beam mentioned above.

A beam splitter, a filter, and the like may be suitably provided between the light beam generator and the optical fiber or in the middle of the optical fiber in order to transmit information to a monitor apparatus or the like that can be included in the apparatus.

The light-emitting window 2 emits a light beam to the abnormal electrical conduction site or site at which hyperexcitability occurs, the abnormal electrical conduction site or site at which hyperexcitability occurs is irradiated with a light beam transmitted inside the optical fiber 4, and cells at such site are necrotized. When a tissue in the vicinity of the junction between the pulmonary vein and the left atrium is targeted for the treatment of atrial fibrillation, for example, cells are preferably killed over the whole circumference. That is, the surrounding area of the pulmonary vein is continuously irradiated with a light beam in a linear manner. To this end, the catheter end may be moved in a linear manner while emitting a light beam. The range of the area of the abnormal electrical conduction site or site at which hyperexcitability occurs irradiated with a light beam emitted from an area close to the distal end of the optical fiber 4 is preferably from 0.5 cm² to 3 cm². Even when the range of light irradiation is restricted and narrow, the direction of irradiation can be changed by rotating the catheter 1 or the like depending on the size of the abnormal electrical conduction site or site at which hyperexcitability occurs, and such site can be subjected to light beam irradiation more than once. Thus, a target tissue can be completely destroyed. When the irradiation with a light beam is carried out, cells at a deep site can be necrotized by applying a high-intensity light beam or a low-intensity light beam for a long period of time. The apparatus comprising the catheter of the present invention has transmurality. The term "transmurality" used herein means that the atrial muscle can be treated from the inside to the outside. The distance from the inside to the outside of the atrial muscle is approximately 3 mm to 5 mm. In the case of treatment of atrial fibrillation, for example, it is sufficient to necrotize the abnormal electrical conduction site or site at which hyperexcitability occurs to a depth of 3 mm to 5 mm.

The means that enables monitoring of the concentrations of an agent for photodynamic therapy and oxygen at the abnormal electrical conduction site or site at which hyperexcitability occurs is an apparatus that monitors fluorescence derived from the agent for photodynamic therapy, phosphorescence, or fluorescence derived from oxygen at the abnormal electrical conduction site or site at which hyperexcitability occurs. Such fluorescence or phosphorescence is transmitted back in the optical transmission fiber. In this case, a fiber that has transmitted a laser beam may be used as the fiber for monitoring fluorescence or phosphorescence, or a fiber exclusively used for monitoring may be separately provided in the catheter 1. When a fiber for monitoring fluorescence or phosphorescence is also used as a fiber for transmitting a light beam, the path of fluorescence or phosphorescence is changed by a beam splitter provided between a light beam generator and a light beam emission site, light passes through an appropriate filter, only light with a desired wavelength is selected, and such light then reaches a detector. When the fiber for monitoring fluorescence or phosphorescence is separate from the fiber for transmitting a light beam, the fiber for monitoring fluorescence or phosphorescence is directly connected to the detector, and fluorescence or phosphorescence reaches the detector through the fiber. By analyzing fluorescence or phosphorescence with the detector, the amount of the agent for photodynamic therapy and the oxygen concentration can be monitored. Since a porphyrin ring of an agent for photodynamic therapy emits fluorescence when excited, for example, the amount of an agent for photodynamic therapy can be measured by measuring the fluorescence. Also, phosphorescence extinction occurs depending on oxygen concentration, and oxygen concentration can thus be measured by measuring phosphorescence. Alternatively, an oxidation fluorescence indicator, the fluorescence intensity of which is increased by active oxygen, may be used, or the phenomenon of a ruthenium complex being fixed with an optical fiber or fluorescence reactions of a ruthenium complex disappearing depending on oxygen concentration may be utilized. Local oxygen partial pressure can be measured according to the description in, for example, J. M. Vanderkooi et al., the Journal of Biological Chemistry, Vol. 262, No. 12, Issue of April 25, pp. 5476-5482, 1987, the Chemical Society of Japan (ed.) Experimental Chemistry Course (Spectroscopy II), pp. 275-194, 1998, or Lichini, M. et al., Chem. Commun., 19, pp. 1943-1944, 1999. The detector is electronically connected to the light beam generation means, the amounts of the agent for photodynamic therapy and oxygen accumulated are fed back by the detection means, and such amounts can be controlled in real time by changing the conditions for light beam irradiation, such as light beam intensity and irradiation time, according to need.

### Use of the catheter of the present invention

A catheter may be inserted into the body from the femoral artery or the upper arm artery by a conventional technique. In general, a catheter may be inserted into the right atrium from the femoral vein, so as to reach the left cardiac tissue in a transseptal manner by the Brockenbrough method. Such technique can be performed by inserting the catheter 1 into the heart or an area in the vicinity thereof from the femoral artery, the femoral vein, the upper arm artery, or the upper arm vein, transporting the light beam irradiation site to the abnormal electrical conduction site or site at which hyperexcitability occurs, and irradiating such site with a light beam. Alternatively, open-heart surgery or laparoscopic surgery may be performed, and the abnormal electrical conduction site or site at which hyperexcitability occurs can be irradiated with a light beam using the catheter of the present invention. Methods using the catheter of the present invention in a therapy include, for example, a step of inserting a catheter into a vein or an artery, a step of leading the catheter to the atrium by an appropriate operation through the vein or the artery, a step of leading the catheter to a target region, a step of positioning the apparatus in the target region, a step of allowing the apparatus to emite a light beam toward the target region and release energy, and a step of measuring potential at the target site using the electrodes for measurement. The catheter 1 can be inserted by a conventional method. In such a case, an appropriate guide sheath or guide wire may be used. At such time, an agent for photodynamic therapy is allowed to be present in an abnormal area in advance by administering the above-mentioned water-soluble agent for photodynamic therapy to a subject of treatment via intravenous injection or other means. The target site can be irradiated with a light beam, so as to damage the tissue thereof.

The abnormal site may be irradiated with a light beam continuously in a linear or dotted manner. At such time, it is preferable that a catheter end be bent so as to face the target site and a light beam be emitted toward the direction in which the catheter end faces. In the case of a lateral-light-emission catheter, it is preferable that a light beam be emitted in the direction in which a catheter end is bent; that is, a light beam is emitted laterally from the inside of the catheter. When atrial fibrillation is to be treated, it is preferable that a light beam be continuously emitted in a linear manner to perform photodynamic ablation by electrical pulmonary vein (PV) isolation.

After the target site of cardiac muscle tissue is irradiated with a light beam from the light-emitting window, potential is measured at two or more points in the periphery of the region subjected to photodynamic ablation using at least two electrodes for potential measurement provided in the periphery of the light-emitting window, and potential differences between arbitrary points are measured. Measurement of potential differences enables the determination of whether or not new tissue cells in the region subjected to photodynamic ablation were necrotized; that is, whether or not photodynamic ablation was satisfactorily performed.

The present invention is described in detail with reference to the examples below, although the technical scope of the present invention is not limited thereto.

### <Example 1: Electrical conduction blocking via early photodynamic therapy using cardiac muscle tissue extirpated from a rat (ex vivo)>

The right ventricular tissue isolated from a Wistar rat was used as a sample. Talaporfin sodium was used as a photosensitive pigment. Talaporfin sodium was dissolved in a perfusion fluid at 4.3 µg/ml. Tyrode's solutions were used as perfusion fluids (95% CO₂, 5% O₂ 37°C). A semiconductor laser with a central wavelength of 670.8 nm was used as an excitation light source, and the irradiation was carried out at 150 mW/cm² and 3.5 J/cm².

An experiment of electric conduction blocking in the cardiac muscle was carried out by the following procedures.
1. The heart was extirpated under deep anesthesia and the right ventricular wall was excised.
2. The excised tissue was subjected to perfusion in a Tyrode's solution comprising talaporfin sodium dissolved therein for 2 hours.
3. Three bipolar electrodes were positioned in cardiac muscle tissue. One such electrode was used for electrical stimulation (for conductance) and the two other electrodes were used for potential measurement (A and B).
4. A band-like laser beam was irradiated to the region between electrodes for potential measurement to perform photodynamic therapy.
5. Potentials at the electrodes for potential measurement A and B were measured during laser irradiation.

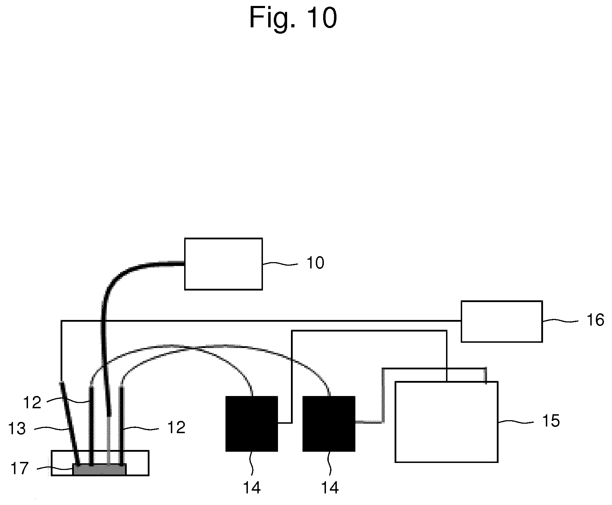

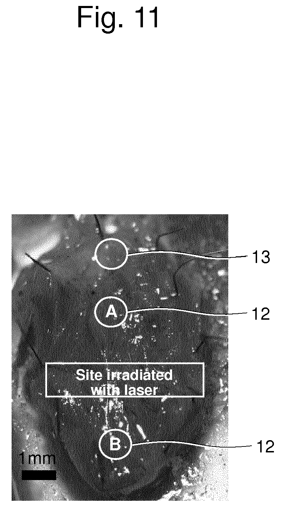

Fig. 10 shows an *ex vivo* experimental system. Fig. 11 shows the positional relationship among the electrically-stimulated site, the electrodes for potential measurement, and the site irradiated with light in cardiac muscle tissue extirpated from a rat.

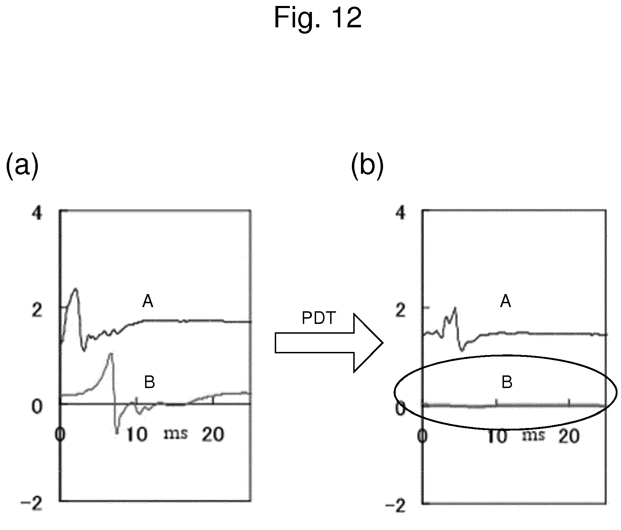

Fig. 12(a) and Fig. 12(b) each show a waveform of transmitted stimulus measured in regions A and B before photodynamic therapy and 5 minutes after photodynamic therapy. The results shown in the figures demonstrate the following. Electrical stimulus was conducted from region A to region B before photodynamic therapy. However, electric conductivity of cardiac muscle tissue was lost at the site irradiated with light after photodynamic therapy, region A was electrically isolated from region B, and electrical signals were not measured at region B.

### <Example 2: Acute experiment (open-heart surgery) using swine: verification of immediate conduction block>

Left auricular tissue obtained from a swine (body weight: 15.4 kg) was used as a sample. Talaporfin sodium was used as a photosensitive dye, and it was administered intravenously to the swine at 10 mg/kg of the body weight. The duration until light irradiation after the administration of the photosensitive dye was 30 minutes. Light irradiation was carried out using a semiconductor laser with a central wavelength of 663 nm at a power density of 5.2 W/cm², an energy density of 208 J/cm², and a spot size of 7 mm Φ.

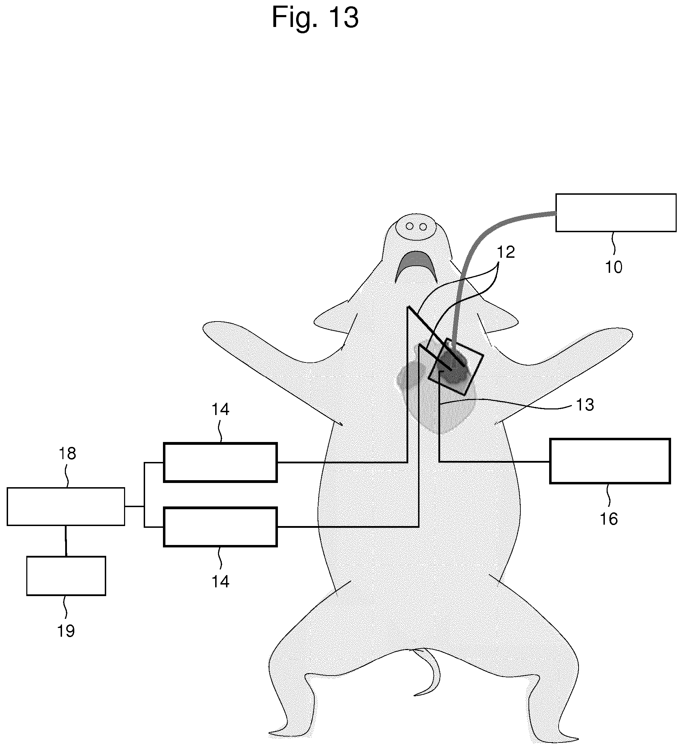

Fig. 13 shows an experimental system using swine.

The experiment for verification of immediate conduction block using swine was carried out in accordance with the following procedure.
1. A swine was subjected to open-heart surgery under deep anesthesia and the left auricle was exposed.
2. Three bipolar electrodes were positioned in cardiac muscle tissue of the left auricle. One such electrode was used for electrical stimulation (for conductance) and the two other electrodes were used for potential measurement (A and B).
3. Talaporfin sodium was administered intravenously at 10 mg/kg.
4. The irradiation with a band-like laser beam was carried out in a dotted manner between electrodes for potential measurement 30 minutes after administration to perform photodynamic therapy.
5. The potential of an electrode for potential measurement B was measured during laser beam irradiation.
6. Loss of conductivity was confirmed based on a delay in electrical signal conductivity.

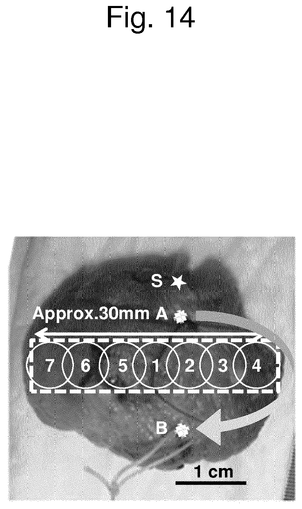

Fig. 14 shows positions of the site irradiated with light and electrodes. In the figure, references A and B each independently represent an electrode for potential measurement, and S represents an electrode for stimulation (i.e., a conducting electrode). In the figure, numeral references 1 to 7 indicate regions irradiated with laser beams. Region 1 to region 7 were irradiated with laser beams in such order. The length of the region irradiated was approximately 30 mm.

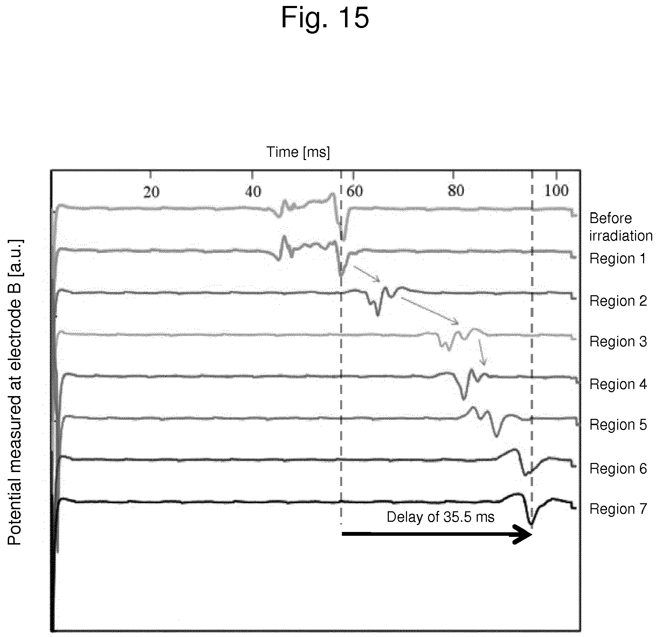

Fig. 15 shows the results. The line at the top indicates the electric potential waveform at the electrode B for measurement before light irradiation, and the other lines each indicate the electric potential waveform at the electrode B for measurement after light irradiation at region 1 to region 7 in the order from top to bottom. The electric potential waveform is obtained by measuring potential with reference to that of the counter electrode A. Electricity conducted from the electrode S for stimulation is transmitted, and it can be measured as a change in the electric potential waveform at electrode B. Prior to laser beam irradiation, cardiac muscle tissue between the electrode S for stimulation and the electrode B for measurement is not necrotized by photodynamic ablation, and electricity is conducted linearly from the electrode S for stimulation to the electrode B for measurement. Once cardiac muscle tissue between the electrode S for stimulation and the electrode B for measurement is necrotized by photodynamic ablation conducted via laser beam irradiation, however, electricity is not conducted in the necrotized region. As the area of the necrotized region increases, accordingly, delay is observed in electric conduction at the electrode B for measurement more frequently since electricity is conducted while bypassing the outside of the necrotized region. As shown in the figure, electric conduction was delayed by approximately 35.5 ms due to the formation of a light irradiation line of approximately 30 mm before laser beam irradiation. Thus, the acute disappearance of electric conductivity by photodynamic therapy was verified.

### Industrial Applicability

The catheter of the present invention can be used for the treatment of arrhythmia such as atrial fibrillation.

### Description of numeral references

1: Catheter
2: Light-emitting window
3: Electrode
4: Optical fiber
5: Mirror
6a: Prism
6b: GRIN lens
7: Marker for monitoring direction of light emission
8: Laser beam
9: Catheter apparatus
10: Laser beam generating apparatus
11: Potential measuring instrument
12: Electrode for potential measurement
13 Electrode for stimulation (conducting electrode)
14 Amplifier
15 Ocilloscope
16 Stimulator
17 Cardiac muscle tissue
18: Signal recorder
19: Computer

## Claims

1. A catheter (1) used for performing photodynamic ablation of cardiac muscle tissue via photochemical reactions in the blood vessel or cardiac lumen, the catheter comprising:
a light-emitting window (2) for irradiating the target site of cardiac muscle tissue with a light beam transmitted through an optical fiber (4) and at least two electrodes (3) for potential measurement located in the periphery of the light-emitting window so as to sandwich the same, so that potential at sites on both sides of cardiac muscle tissue that has been subjected to ablation with the light beam emitted from the light-emitting window and potential differences between such sites can be measured, wherein a cylindrical light-emitting window with a spherical end is provided at a catheter end, the light beam transmitted through an optical fiber is emitted from the light-emitting window coaxially with a longitudinal axis of the catheter, and wherein at least two planar electrodes for potential measurement are provided in the periphery of the light-emitting window.

2. The catheter according to claim 1, comprising an end which is freely bendable.

3. The catheter according to claim 1 or 2, wherein the light-emitting window has a ring or cylinder shape.

4. The catheter according to any one of claims 1 to 3, which comprises at least one construct configured to reflect the light beam transmitted through an optical fiber in a lateral direction.

5. The catheter according to claim 4, wherein the at least one construct is one of a mirror (5), prism (6a), lens (6b) or a combination of two or more thereof.

6. The catheter according to any one of claims 1 to 5, which further comprises:
at least one marker (7) for monitoring the direction of an emitted light beam from outside of a body in the vicinity of the distal end of the catheter, wherein the position or configuration of the marker is associated with the direction of the irradiation of a light beam from the catheter.

7. The catheter according to claim 6, wherein the at least one marker comprises markers provided asymmetrically relative to the longitudinal axis of the catheter.

8. The catheter according to claim 6 or 7, wherein the at least one marker is in the form of a line, ribbon, or ring and is configured to cross the longitudinal axis of the catheter along the outer periphery of the distal end of the catheter.

9. The catheter according to any one of claims 6 to 8, wherein the at least one marker is a radiopaque marker.

10. The catheter according to any one of claims 6 to 9, wherein the at least one marker serves as an electrode for potential measurement (12).

11. The catheter according to any one of claims 1 to 10, wherein the light beam is a laser or LED light.

12. A photodynamic ablation catheter apparatus configured to block abnormal electrical conduction of the cardiac muscle by conducting photodynamic therapy using an agent for photodynamic therapy, the apparatus comprising:
the catheter according to any one of claims 1 to 11,
a means for generating the light beam (10) configured to irradiate an abnormal electrical conduction site or hyperexcitability site at an excitation wavelength of the agent for photodynamic therapy, and
a means for transmitting the light beam to the abnormal electrical conduction site.

## Patentansprüche

1. Katheter (1) zur Verwendung zum Durchführen einer fotodynamischen Ablation von Herzmuskelgewebe über fotochemische Reaktionen in dem Blutgefäß oder dem Herzlumen, wobei der Katheter Folgendes umfasst:
ein lichtemittierendes Fenster (2) zum Bestrahlen der Zielstelle des Herzmuskelgewebes mit einem Lichtstrahl, der durch eine optische Faser (4) übertragen wird und zumindest zwei Elektroden (3) für eine Potential-Messung, die sich in der Peripherie des lichtemittierenden Fensters befinden, um dasselbe sandwichartig zu umgeben, so dass Potential an Stellen auf beiden Seiten des Herzmuskelgewebes, das der Ablation mit dem Lichtstrahl, der aus dem lichtemittierenden Fenster emittiert wurde, unterzogen wurde und Potentialunterschiede zwischen solchen Stellen gemessen werden können, worin ein zylindrisches lichtemittierendes Fenster mit einem kugelförmigen Ende an einem Katheter-Ende bereitgestellt ist, wobei der Lichtstrahl, der durch eine optische Faser übertragen wird, aus dem lichtemittierenden Fenster koaxial mit einer Längsachse des Katheters emittiert wird und worin zumindest zwei Planarelektroden für eine Potential-Messung in der Peripherie des lichtemittierenden Fensters bereitgestellt sind.

2. Katheter nach Anspruch 1, der ein Ende umfasst, das frei biegbar ist.

3. Katheter nach Anspruch 1 oder 2, worin das lichtemittierende Fenster eine Ring- oder eine Zylinderform aufweist.

4. Katheter nach einem der Ansprüche 1 bis 3, der zumindest ein Konstrukt umfasst, das konfiguriert ist, den Lichtstrahl, der durch eine optische Faser übertragen wird, in eine laterale Richtung zu reflektieren.

5. Katheter nach Anspruch 4, worin das zumindest eine Konstrukt eines aus einem Spiegel (5), einem Prisma (6a), einer Linse (6b) oder einer Kombination aus zwei oder mehr davon ist.

6. Katheter nach einem der Ansprüche 1 bis 5, der ferner Folgendes umfasst:
zumindest eine Markierung (7) zum Überwachen der Richtung eines emittierten Lichtstrahls von außerhalb eines Körpers in der Umgebung des distalen Endes des Katheters, worin die Position oder die Konfiguration der Markierung der Richtung der Bestrahlung eines Lichtstrahls aus dem Katheter zugeordnet ist.

7. Katheter nach Anspruch 6, worin die zumindest eine Markierung Markierungen umfasst, die in Bezug auf die Längsachse des Katheters asymmetrisch bereitgestellt sind.

8. Katheter nach Anspruch 6 oder 7, worin zumindest eine Markierung die Form einer Linie, eines Bands oder eines Rings aufweist und konfiguriert ist, die Längsachse des Katheters entlang der äußeren Peripherie des distalen Endes des Katheters zu schneiden.

9. Katheter nach einem der Ansprüche 6 bis 8, worin die zumindest eine Markierung eine strahlenundurchlässige Markierung ist.

10. Katheter nach einem der Ansprüche 6 bis 9, worin die zumindest eine Markierung als eine Elektrode für eine Potential-Messung (12) dient.

11. Katheter nach einem der Ansprüche 1 bis 10, worin der Lichtstrahl ein Laser- oder LED-Licht ist.

12. Fotodynamische Ablationskathetervorrichtung, die konfiguriert ist, eine anomale elektrische Leitung des Herzmuskels durch Durchführen einer fotodynamischen Therapie unter Verwendung eines Mittels zur fotodynamischen Therapie zu blockieren, wobei die Vorrichtung Folgendes umfasst:
den Katheter nach einem der Ansprüche 1 bis 11,
ein Mittel zum Erzeugen des Lichtstrahls (10), der konfiguriert ist, eine anomale elektrische Leitungsstelle oder Übererregbarkeitsstelle bei einer Anregungswellenlänge des Mittels zur fotodynamischen Therapie zu bestrahlen, und
ein Mittel zum Übertragen des Lichtstrahls an die anomale elektrische Leitungsstelle.

## Revendications

1. Cathéter (1) utilisé pour réaliser une ablation photodynamique de tissu musculaire cardiaque via des réactions photochimiques dans le vaisseau sanguin ou la lumière cardiaque, le cathéter comprenant :
une fenêtre émettrice de lumière (2) pour rayonner le site cible du tissu musculaire cardiaque avec un faisceau lumineux transmis par le biais d'une fibre optique (4) et au moins deux électrodes (3) de mesure de potentiel se trouvant à la périphérie de la fenêtre émettrice de lumière de sorte à la prendre en sandwich, afin que le potentiel au niveau des sites sur les deux côtés du tissu musculaire cardiaque qui a été soumis à ablation avec le faisceau lumineux émis à partir de la fenêtre émettrice de lumière et les différences de potentiel entre ces sites peuvent être mesurées, dans lequel
une fenêtre émettrice de lumière cylindrique ayant une extrémité sphérique est prévue au niveau d'une extrémité de cathéter, le faisceau lumineux transmis par l'intermédiaire d'une fibre optique est émis à partir de la fenêtre émettrice de lumière coaxialement avec un axe longitudinal du cathéter, et dans lequel au moins deux électrodes planes de mesure de potentiel sont prévues à la périphérie de la fenêtre émettrice de lumière.

2. Cathéter selon la revendication 1, comprenant une extrémité qui peut être fléchie librement.

3. Cathéter selon la revendication 1 ou 2, dans lequel la fenêtre émettrice de lumière a une forme annulaire ou cylindrique.

4. Cathéter selon l'une quelconque des revendications 1 à 3, qui comprend au moins une construction configurée pour réfléchir le faisceau lumineux transmis à travers une fibre optique dans une direction latérale.

5. Cathéter selon la revendication 4, dans lequel l'au moins une construction est l'une d'un miroir (5), d'un prisme (6a), d'une lentille (6b) ou d'une combinaison de deux ou plusieurs d'entre eux.

6. Cathéter selon l'une quelconque des revendications 1 à 5, qui comprend en outre :
au moins un marqueur (7) pour surveiller la direction d'un faisceau lumineux émis depuis l'extérieur d'un corps au voisinage de l'extrémité distale du cathéter, dans lequel la position ou la configuration du marqueur est associée à la direction du rayonnement d'un faisceau lumineux à partir du cathéter.

7. Cathéter selon la revendication 6, dans lequel l'au moins un marqueur comprend des marqueurs prévus de manière asymétrique par rapport à l'axe longitudinal du cathéter.

8. Cathéter selon la revendication 6 ou 7, dans lequel l'au moins un marqueur est sous la forme d'une ligne, d'un ruban ou d'un anneau et est configuré pour croiser l'axe longitudinal du cathéter le long de la périphérie externe de l'extrémité distale du cathéter.

9. Cathéter selon l'une quelconque des revendications 6 à 8, dans lequel l'au moins un marqueur est un marqueur radio-opaque.

10. Cathéter selon l'une quelconque des revendications 6 à 9, dans lequel l'au moins un marqueur sert d'électrode pour la mesure de potentiel (12).

11. Cathéter selon l'une quelconque des revendications 1 à 10, dans lequel le faisceau lumineux est une lumière laser ou DEL.

12. Appareil à cathéter d'ablation photodynamique configuré pour bloquer la conduction électrique anormale du muscle cardiaque en opérant une thérapie photodynamique au moyen d'un agent de thérapie photodynamique,
l'appareil comprenant :
le cathéter selon l'une quelconque des revendications 1 à 11,
des moyens pour générer le faisceau de lumière (10) configurés pour rayonner un site de conduction électrique anormale ou un site d'hyperexcitabilité à une longueur d'onde d'excitation de l'agent de thérapie photodynamique, et
des moyens pour transmettre le faisceau lumineux au site de conduction électrique anormale.
